# EUROPEAN PATENT SPECIFICATION

(11) **EP 0 542 526 B1**
(45) Date of publication and mention of the grant of the patent: **23.04.1997**
(21) Application number: 92310295.8
(22) Date of filing: 11.11.1992
(51) Int. Cl.: A61K 7/50

(54) **Skin cleansing compositions**
Hautreinigungsmittel
Compositions de nettoyage de la peau

(30) Priority: 12.11.1991 US 791762
(43) Date of publication of application: 19.05.1993
(73) Proprietor: UNILEVER PLC, London EC4P 4BQ (GB); UNILEVER N.V., 3013 AL Rotterdam (NL)
(72) Inventor: Rys-Cicciari, Karla Jean, NL-2651 ET Berkel en Rodenrijs (NL); Greene, Alan Paul, Flemington, New Jersey 08822 (US); Osmer, Frederick Sylvio, Parsippany, New Jersey 07054 (US); Ashley, Jeanette Frances, Verona, New Jersey 07044 (US); Lee, Robert Stanley, Spital, Wirral L63 9LD (GB); Coxon, Andrew Charles, Wirral, Merseyside L62 6EP (GB); Podgorsky, Joseph James, Slate Hill, New York 10973 (GB); Rerek, Mark Edward, Woking, Surrey GU22 0NX (GB)
(74) Representative: Elliott, Peter William

(56) References cited:
- WO-A-90/13283
- WO-A-92/08440
- GB-A- 2 245 281

## Description

### FIELD OF THE INVENTION:

The present invention relates to skin cleansing compositions containing major amounts of acyl isethionates together with other detergent.

### PRIOR ART:

Traditionally, soap has been utilised as a skin cleanser. Most modern soaps comprise a mixture of soaps obtained from coconut fats and tallows. The lauric acid soaps and other soluble soaps derived from the coconut fats contribute the lathering properties of the overall composition, whereas the longer chain soaps obtained from the tallows provide structure. Soap is, however, a very harsh chemical. Irritated and cracked skin result from use of soap, especially in colder climates. There are, however, certain benefits from the use of soap including low cost, ease of manufacture into bars, and good lathering properties.

There has been much commercial activity in attempting to replace soaps with milder surfactants. The introduction of mild synthetic detergent toilet bars, especially those based on sodium cocoyl isethionates, has been particularly successful.

Sodium cocoyl isethionate is a mixture of compounds having the general formula:

CH₃-(CH₂)ₙ-CO-OCH₂CH₂SO₃Na

wherein the value of n lines in the range of alkyl chain lengths found in the fatty acids derived from coconut fats

Patents relating to this technology are fully discussed in granted U.S.Patent No.4954282, which is incorporated by reference herein. From this prior art it will be seen that many isethionate derivatives are known in the art of making skin cleansing compositions such as toilet bars.

Further developments in this field are discussed in Applicants own European patent application EP-A-472320 published 26 February 1992. That application discloses compositions with excellent skin mildness which also maintain good lather properties: these comprise selected acyl esters of isethionic acid salts in an amount of about 20 to 70% by weight and at least one betaine wherein the weight ratio of said acyl esters to betaine is about 10:1 to about 2:1. The selected acyl esters of isethionic acid disclosed in EP-A-472320 have a chain length distribution as shown in Table 1 below.

**TABLE 1**

| Chain Length | Wt. % Fatty Acid Combined As Cocoyl Isethionate |
|---|---|
| C₆-C₁₀ | 10-25 |
| C₁₂ | 45-55 |
| C₁₄ | 20-40 |
| C₁₈ unsaturated | 1-15 |

It is known from EP-A-0472320 to be desirable for the selected isethionate based esters to be combined with selected betaines to provide mildness to the skin together with good bar properties such as good lather volume.

The general class of betaines can be subdivided into a number of narrower sub-classes of compound. These include simple betaines of the general formula: 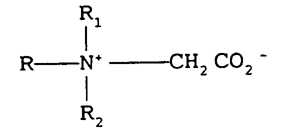 wherein R is long chain alkyl and R₁ and R₂ are generally short chain alkyl or hydroxy alkyl.

In addition to the simple betaines, a number of derivatives are known in which linker groups are inserted between the quaternary nitrogen and the primary alkyl chain, R, or in which the carboxyl group is replaced by an alternative 'acidic' group. These classes include the amidopropyl betaines and the sulpho-betaines (hydroxy sultaines) respectively.

Betaines and amidobetaines are known to be very mild to both skin and eyes. Cocamido-betaines in which the group R has a chain length distribution similar to that of the fatty acids in coconut oil (as described above for cocoyl isethionates), in particular, have good lather properties.

Alternatives to betaine are known. Amongst these are the sulphosuccinates. Generally, alkyl sulphosuccinate esters comply with one or other of the general formulae: 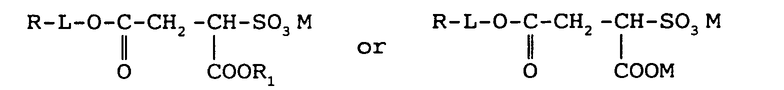 wherein R and R₁ are alkyl, L either is absent or is a linking group such as -CO-NH-CH₂-CH₂-, or -(OC₂H₄)ₙ-, and M is a cation, such as sodium.

The sulphosuccinates may be further divided into the classes of amido sulphosuccinates and alkyl sulphosuccinates. In simple alkyl sulphosuccinates, the linking group is absent. In the amido sulphosuccinates the linking group includes an amide group and usually is -CONHCH₂CH₂ - as specified above.

The Applicants own European published application EP-A-441652, published on 14 August 1991 relates to a skin cleansing composition which includes a combination of acyl isethionate ester salts and at least one sulphosuccinate. In the embodiments described, the weight ratio of acyl isethionate to sulphosuccinate is about 10:1 to about 2:1. The particular sulphosuccinates which are disclosed as preferred components in the prior application are the amido sulphosuccinates as specified above.

WO 92/08440, published on 29 May 1992 includes an Example 7 which mentions a composition which was an aqueous liquid containing 15% by weight of a detergent mixture consisting of cocoyl isethionate, coconut amidopropyl betaine and disodium lauryl ether sulphosuccinate with average 3 ethylene oxide residues, these three detergents being in a ratio of 50:40:10 by weight.

### THE INVENTION:

The present invention provides a skin cleansing composition comprising: acyl isethionate (i.e. acyl esters of isethionic acid salts), at least one sulphosuccinate and at least one betaine.

Preferably the composition is in the form of a bar although liquid compositions are not excluded.

The mildness of a composition can be enhanced, in accordance with this invention, by the incorporation of both sulphosuccinate and betaine along with acyl isethionate. Another advantage of the invention is that the ease of processing the composition is improved by the incorporation of sulphosuccinate as well as betaine. In consequence it is possible to obtain a very satisfactory combination of mildness and processability while keeping the total quantity of co-active (betaine plus sulphosuccinate) down to a moderately low level.

The cleansers resulting from this mixture of actives have superior skin mildness, excellent lather and good tactile characteristics. In addition, they are easily processable using standard manufacturing equipment.

The isethionate ester salts that may be employed herein are acyl isethionates, i.e. acyl esters of isethionic acid salts, with an anion of the general formula

R-CO-O-CH₂CH₂SO₃ -

wherein R-CO- is an aliphatic acyl group with a hydrocarbon chain length distribution such that at least 90% of the acyl groups R-CO- contain from 6 to 18 carbon atoms. The accompanying cation M⁺ may be alkali metal, especially sodium, ammonium or substituted ammonium.

Preferred is cocoyl isethionate which may be prepared from the usual cocoyl fatty acids having a small percentage of fatty acid chains below C₈ with over 95% of the carbon chain distribution being between C₈ and C₁₈ and more than half being C₁₂ or less. The acyl chain length of these preferred esters will have at least about 90% C₆-C₁₈ and more than about 30% C₁₄ or lower.

Compositions embodying the present invention preferably contain 20-70% by weight of acyl isethionate. Amounts of 40-60% by weight are particularly preferred. Preferably the ratio of acyl isethionates to other detergent actives is in a range from 10:1 to 2:1.

In addition to acyl isethionate it is preferable that the formulations free isethionate, preferably as the sodium or other alkali metal salt. Typically, the amount of free isethionate (if any) in embodiments of the invention range from 1-10% by weight of the composition and more preferably 3-7% by weight of the composition.

The betaines to be used in the invention have the following structure (I): 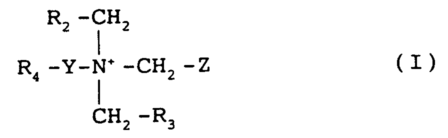 where R₄ is a preferably aliphatic hydrocarbon chain having a chain length distribution such that at least 90% of the R₄ groups contain 5 to 17 carbon atoms,
R₂ and R₃ are independently hydrogen, alkyl of 1 to 4 carbon atoms or hydroxyalkyl of 1 to 4 carbon atoms such as CH₂OH,
Y is CH₂ or of the form CONHCH₂CH₂CH₂ (amidopropyl betaine); Z is either a COO⁻ (carboxybetaine), or of the form CHOHCH₂SO₃- (sulphobetaine or hydroxy sultaine).

If R₂ and R₃ are both hydrogen while Y and Z are repsectively CH₂ and COO⁻, it is preferred that R₄ is less than 50% C₁₅-C₁₇.

Preferably to ensure processability along with mildness and other use properties the betaine is an amidopropyl betaine.

Specific betaines useful in the invention are lauryl betaine (Varion CDG from Sherex), cocamidopropyl betaine (Varion CADG from Sherex), coco betaine (Mackam CB from McIntyre), cocamidopropyl hydroxy sultaine (Varion CAS from Sherex) and tallow dihydroxyethyl glycinate (Varion TEG from Sherex).

Particularly preferred is cocoamidopropyl betaine of the structure II:

R-CO-NH-CH₂-CH₂-CH₂-N⁺(CH₃)₂.CH₂-COO⁻ (II)

where R is derived from coconut fatty acids, although in other betaines it may be any convenient, especially alkyl group. The chain length distribution of such coconut fatty acids will be similar to that for the coco group on the isethionate. The chain length distribution will contain at least 90% of C₈ to C₁₈ with more than half made up of C₈ to C₁₄. A typical fatty acid distribution in the cocoyl portion is the same as in Table 1.

Compositions according to the present invention preferably contain from 0.5 to 10% or from 1 to 10% by weight of the betaine.

Sulphosuccinates useful in the present invention may be various esters including both monoesters and diesters. The general formula for this anion is 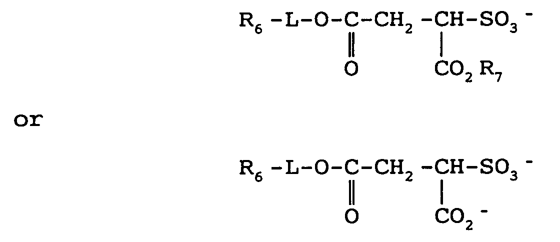 where R₆ and R₇ if present are independently hydrocarbon chains showing a chain length distribution such that at least 90% of each of the R₆ and R₇ groups contains 6 to 18 carbon atoms and L is absent or is a linking group.

Accompanying cations may be alkali metal, especially sodium, ammonium or substituted ammonium.

The preferred sulphosuccinates are those prepared through an alkanolamine intermediate so as to introduce an amido group into the molecule.

Particularly preferred is cocamido monoethanolamine sulphosuccinate of the structure (III) 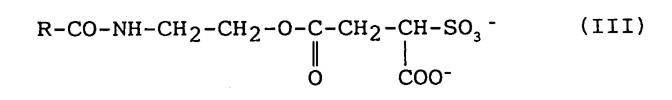 R in Structure (III)is derived from coconut fatty acids, although in other sulphosuccinates it may be any convenient alkyl group. The chain length distribution of such coconut fatty acids will be similar to that for the coco group on the isethionate. The chain length distribution will contain at least 90% of C₈ to C₁₈ with more than half made up of C₈ to C₁₄. A typical fatty acid distribution in the cocoyl portion is the same as in Table 1.

The preparation of the abovementioned compound (III) is described in EP-A-0441652 mentioned above.

Compositions according to the present invention preferably contain from 1 to 24%, more preferably from 1 to 10% by weight of the sulphosuccinate.

It is preferable, in embodiments of the present invention, that particular ratios of betaine to sulphosuccinate are selected. In particular, the ratio preferably falls in the range 2:1 to 1:10, better 2:1 to 1:7. It may lie in the range 1:1 to 1:7, preferably 1:1 to 1:6, most preferably 1:2 to 1:4.

As previously noted, soap may be somewhat harsh and when present in the compositions of this invention should desirably be at a level no higher than about 35% by weight, preferably less than 5%, and advantageously totally absent.

Free fatty acids of about 8-22 carbon atoms are desirably incorporated within the compositions of the present invention. Some of these fatty acids are present to operate as superfatting agents and others as skin feel and creaminess enhancers. Fatty alcohols, fatty amides and the like may also be employed. Superfatting agents enhance lathering properties and may be selected from fatty acids with 8 to 18 carbon atoms, preferably 10-10 carbon atoms, in an amount up to 40% by weight of the composition. Skin feel and creaminess enhancers, the most important of which is stearic acid, are also desirably present in these compositions.

Other chemicals and adjuncts may be employed with these compositions. The amount of these chemicals and adjuncts may range from about 0% to 20% by weight of the total compositions. For instance, there may be included humectants such as glycerine; anti wear agents such as polymer JR and natural and synthetic gums and the like; germicides, perfumes, colourants, dyes, pigments such as titanium dioxide, electrolytes and water. It is also preferable to include metal sequestering agents such as ethylene diamine tetraacetic acid (EDTA).

Evaluation of skin mildness properties were carried out by means of a standardised Flex Wast Test in which a product's skin mildness properties were evaluated in a paired comparison with a control product. The procedure is as follow:

The Flex Wash test procedure consists of three daily two minute washes of the antecubital fossa (flex area of elbow). This method is an "exaggerated use" method designed to differentiate very mild products. Erythemal response varies only slightly with temperature and humidity fluctuations making the protocol suitable for year round testing.

Approximately 15 panellists were used as the test population. Panellist flex areas must be free of any skin condition (eczema, dryness, irritation, cuts or abrasions). Anyone taking antihistamines, anti-inflammatory drugs (more than 8 per week) or topical, oral or injectable cortisone or a regular basis was excluded from the study. The panel was divided into two sub-groups which were balanced for left handedness. Group I was assigned the test product for the left flex area and the control for the right flex area. Group 11 reversed the order.

Following an evaluation, the panellist was instructed to moisten the left flex area, the sponge and test compositions formulated as toilet bars were dampened with tap water (100 ppm calcium/ magnesium ions). The sponge was then stroked over the test bar 10 times by the evaluator. The "dosed" sponge was placed in the panellist's right hand. The panellist then washed the left flex area for exactly two minutes. Thereupon, the flex was rinsed and patted dry. This washing procedure was repeated on the right arm with appropriate composition. Thus, both arms are tested simultaneously. Washing by this procedure was repeated three times daily for 5 consecutive days for a total of 15 washes. Treatment times were scheduled 1.5 hours apart. Each test site was evaluated immediately prior to washing and 4 hours after the third daily wash. A slightly different procedure was used for Example 7. In this evaluation, the flex area was only washed for one minute, repeated four times daily for five consecutive days for a total of 20 washes. The test site was evaluated immediately prior to each wash.

One trained assessor evaluated test sites for a total of 20 evaluations. The grading scale was as follows:
- 0: - no erythema
- 0.5: - barely perceptible erythema
- 1: - mild spotty erythema/no edema
- 1.5: - mild/moderate erythema/with or without edema
- 2: - moderate confluent erythema/with or without edema or vesiculation

Each test site was treated in the prescribed method until a grading of "2" or greater was attained or 15 washings had been completed. When a score of "2" or greater was attained, the treatment was discontinued on that flex. The final score was then carried through for all remaining evaluations. The remaining flex was washed until either a grading of at least "2" or 15 treatments were attained, whichever was first. In the Examples of this specification, the final grading, Mean Rank Scores, is the sum total of grade scores for 15 assessments per panellist averaged over the scores from all panellists. Thus, theoretically, the average score could range from 0 to 30; the lower score indicating absolutely no skin irritation while the 30 score being the most severe. Mean Endpoint Erythema scores are the mean of the evaluation scores, for each panellist, at which the first arm received a grade of "2" or greater erythema score or at the completion of fifteen washes. The Mean Rank Erythema scores are analysed and compared using the Wilcoxon Signed Rank Test (two-sample).

The statistical test employed in the comparisons mentioned below derives the 'p-value' for comparisons of formulations according to the invention with controls. This is the probability that the two results being compared are different due to chance as opposed to differences being attributable to real differences. The lower the p-value, the less likely that the results differ due to chance. Thus, a p-value of 0.10 indicates that there is only a 10% chance that the difference between the example and the control is due to chance. P-values of, say, 0.04 indicate that the probability that the difference in results is due to chance is around 4%.

The products' skin mildness properties were evaluated by comparison to a control formulation 'A', which is identical to that of a commercially available bar containing approximately 50% sodium cocoyl isethionate, approximately 23% free fatty acid and approximately 8% soap but neither betaine nor sulphosuccinate.

In addition, comparative trials were performed against the commercially available 'Oil of Olay' [RTM] soap bar, designated 'O' in the examples.

### EXAMPLES:

The following examples will more fully illustrate the embodiments of this invention, and some comparative formulations in trials against compositions 'A' and 'O'. All parts, percentages and proportions referred to herein and in the appended claims are by weight of the total composition unless otherwise stated.

Formulations embodying the invention are set out in the following Table.

| Component | Formulation (% by weight) | | | | | | |
|---|---|---|---|---|---|---|---|
| | 1 | 2 | 3 | 4 | 5 | 6 | 7 |
| Na Cocoyl Isethionate | 47.0 | 47.0 | 47.0 | 50.0 | 48.8 | 38.9 | 50.0 |
| Free fatty acid | 33.75 | 33.75 | 33.75 | 24.42 | 23.8 | 23.5 | 26.2 |
| Na Isethionate | 5.2 | 4.71 | 5.12 | 5.53 | 5.53 | 8.7 | 6.2 |
| Disodium Cocamido(MEA) Sulphosuccinate | 6.0 | 1.0 | 3.5 | 6.0 | 6.0 | 10.0 | 4.5 |
| Cocamidopropyl betaine | 1.0 | 6.0 | 3.5 | 2.0 | 2.0 | 5.9 | 2.0 |
| Soap | - | - | - | - | 2.0 | - | 2.0 |
| PEG 150 | - | - | - | - | - | 3.0 | - |
| Dextrin | - | - | - | - | - | 10.0 | - |
| Sodium Chloride | 0.5 | 1.0 | 1.0 | 0.5 | 1.0 | 1.0 | 0.5 |
| Titanium Dioxide | 0.5 | 0.5 | 0.5 | 0.5 | 0.5 | 0.5 | 0.5 |
| Perfume | 1.0 | 1.0 | 1.0 | 1.0 | 1.0 | 1.0 | 1.0 |
| Cetyl Alcohol | - | - | - | 5.0 | 5.0 | - | 3.0 |
| Miscellaneous | 0.4 | 0.4 | 0.4 | 0.4 | 0.4 | 0.4 | 0.04 |
| Water | balance to 100% | | | | | | |

### Comparisons:

Some comparative formulations are as follows:

| Component | Formulation (% by weight) | | | | | |
|---|---|---|---|---|---|---|
| | 8 | 9 | 10 | 11 | 12 | 13 |
| Na Cocoyl Isethionate | 33.1 | 49.8 | 54.0 | 46.5 | 43.9 | 46.1 |
| Free fatty acid | 29.8 | 23.9 | 15.0 | 32.7 | 20.1 | 23.7 |
| Na Isethionate | 9.1 | 7.7 | 5.0 | 8.2 | 11.0 | 3.7 |
| Disodium Cocamido(MEA) Sulphosuccinate | 15.0 | 12.0 | - | - | 15.2 | 9.0 |
| Cocamidopropyl betaine | - | - | 5.9 | 7.4 | - | - |
| Soap | - | - | - | - | - | - |
| PEG 150 | - | - | 3.0 | - | - | 1.0 |
| Dextrin | - | - | 10.0 | - | - | - |
| NaCl | 0.35 | - | 1.0 | 1.4 | 0.35 | 0.35 |
| TiO₂ | 0.35 | - | 0.5 | - | 0.5 | 0.5 |
| Perfume | 1.0 | - | 1.0 | - | 1.0 | - |
| Cetyl Alcohol | - | - | - | - | - | - |
| Miscellaneous | 1.45 | 0.4 | 0.4 | 0.04 | - | 0.04 |
| Water | balance to 100% | | | | | |

The results of testing a number of these Examples and comparative formulations against Control A were as follows:

| F'lation No. | End Point Erythema | | Mean Rank Scores | | p-value |
|---|---|---|---|---|---|
| | F'lation | Control A | F'lation | Control A | |
| 1 | 0.9 | 1.4 | 15.9 | 23.1 | 0.04 |
| 2 | 1.0 | 1.4 | 16.5 | 22.5 | 0.09 |
| 3 | 1.1 | 1.9 | 12.7 | 24.3 | 0.007 |
| 6 | 0.7 | 2.0 | 9.1 | 25.9 | 0.0000 |
| 8 | 0.8 | 1.7 | 10.8 | 24.2 | 0.0002 |
| 9 | 1.3 | 1.8 | 15.2 | 23.8 | 0.01 |
| 10 | 0.7 | 2.0 | 9.1 | 25.9 | 0.0000 |
| 11 | 1.0 | 1.6 | 12.9 | 22.1 | 0.0051 |
| 12 | 0.8 | 1.5 | 12.3 | 22.7 | 0.0018 |
| 13 | 0.8 | 1.3 | 15.7 | 21.3 | 0.10 |

The results of testing against Control O were as follows:

| F'lation No. | End Point Erythema | | Mean Rank Scores | | p-value |
|---|---|---|---|---|---|
| | F'lation | Control O | F'lation | Control O | |
| 4 | 1.3 | 1.7 | 14.39 | 22.61 | 0.002 |
| 5 | 1.2 | 1.7 | 13.16 | 19.84 | 0.04 |
| 7 | 1.2 | 1.7 | - | - | 0.03 |

From the abovementioned results it can be seen that all of the formulations according to the present invention showed a significant improvement when compared against the commercial formulations.

The hardness and consequent ease of processing was also assessed for most formulations. Comparative formulations 8, 9, 12 and 13 containing sulphosuccinate were all very hard. Comparative formulations 10 and 11 which contained betaine without sulphosuccinate were very soft. The formulations 1 to 5 and 7 according to the invention were intermediate in hardness between the very soft and very hard comparative formulations, which is advantageous for processing.

Thus the formulations of the invention have a hardness which is better than the comparative formulations while generally giving a mildness which is as good as, and in certain instances better than, that of the comparative formulations.

## Claims

1. Skin cleansing composition comprising:
a) acyl isethionate whose anion is of the general formula:
R-CO-OCH₂CH₂SO₃⁻
wherein R-CO- is an aliphatic acyl group with a hydrocarbon chain length distribution such that at least 90% of the acyl groups R-CO- contain from 6 to 18 carbon atoms;
b) at least one sulphosuccinate whose anion is of the general formula: 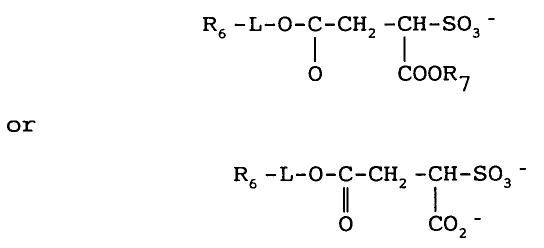 wherein one of R₆ and R₇ are independently aliphatic hydrocarbon chains showing a chain length distribution such that at least 90% of the R₆ or R₇ groups contains 6 to 18 carbon atoms, and
L is absent or is a linking group; and
c) at least one betaine of the general formula: 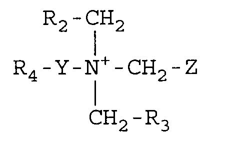 wherein R₄ is an aliphatic hydrocarbon chain having a chain length distribution such that at least 90% of the R₄ groups contain 5 to 17 carbon atoms,
R₂ and R₃ are independently hydrogen, alkyl of 1 to 4 carbon atoms or hydroxyalkyl of 1 to 4 carbon atoms,
Y is CH₂ or CONHCH₂CH₂CH₂, and
Z is CO₂⁻ or CHOHCH₂SO₃⁻,
further characterised in that either
i) L is absent or includes an amido group while R₂ and R₃ are independently hydrogen or -CH₂OH
or
ii) the weight ratio of acyl isethionate to other detergent actives is in a range from 10:1 to 2:1,
but excluding a composition which is an aqueous liquid containing 15% by weight of a detergent mixture consisting of cocoyl isethionate, coconut amidopropyl betaine and disodium lauryl ether sulphosuccinate with average 3 ethylene oxide residues, in weight ratio of 50:40:10.

2. Composition acording to claim 1 in bar form.

3. Composition according to claim 1 in liquid form.

4. Composition according to claim 1 wherein the weight ratio of acyl isethionate to other detergent actives is in a range from 10:1 to 2:1 and the composition is in bar form.

5. Composition according to claim 1 wherein at least 95% of the carbon chain distribution of R lies between C₈ and C₁₈ and more than 30% is C₁₄ or less.

6. Composition according to claim 1 wherein at least 95% of the carbon chain distribution of R lies between C₈ and C₁₈ and more than half is C₁₂ or less.

7. Composition according to any one of the preceding claims wherein the betaine is present in an amount from 0.5 to 10% by weight of the composition, the sulphosuccinate is present in an amount from 1 to 24% by weight of the composition and the acyl esters of isethionic acid salts are present in an amount such that the ratio of acyl isethionates to other detergent actives is in a range from 10:1 to 2:1.

8. Composition according to any one of the preceding claims which further comprises from 1 to 10% by weight of alkali metal isethionate.

9. Composition according to any one of the preceding claims wherein the betaine has the formula:
R₄-CO-NH-CH₂-CH₂-CH₂-N⁺(CH₃)₂.CH₂-COO⁻

10. Composition according to claim 9 wherein at least 95% of the carbon chain distribution of R₄ lies between C₈ and C₁₈ and more than half is C₁₂ or less.

11. Composition according to any one of the preceding claims wherein the amount of betaine is 1-10% by weight of the composition.

12. Composition according to any one of the preceding claims wherein the sulphosuccinate is acyl monoethanolamine sulphosuccinate of the structure:

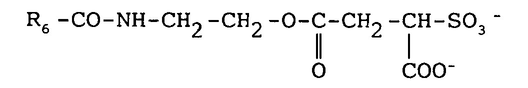

13. Composition according to claim 12 wherein at least 95% of the carbon chain distribution of R₆ lies between C₈ and C₁₈ and more than half is C₁₂ or less.

14. Composition according to any one of the preceding claims wherein the amount of sulphosuccinate is 1-10% by weight of the composition.

15. Composition according to any one of the preceding claims wherein the weight ratio of betaine to sulphosuccinate lies in the range 2:1 to 1:10.

16. Composition according to any one of the preceding claims wherein the weight ratio of betaine to sulphosuccinate lies in the range 1:1 to 1:7.

17. Composition according to any one of the preceding claims which does not contain more than 35% by weight soap.

18. Composition according to any one of the preceding claims, further comprising free fatty acids of about 8-22 carbon atoms.

19. Composition according to any one of the preceding claims wherein R₂ and R₃ are independently hydrogen or -CH₂OH.

## Patentansprüche

1. Hautreinigungszusammensetzung, die die folgenden Bestandteile umfaßt:
a) Acylisethionat, dessen Ariion die folgende Formel besitzt:
R-CO-OCH₂CH₂SO₃⁻
worin R-CO- für eine aliphatische Acylgruppe mit einer derartigen Kohlenwasserstoffkettenlängenverteilung steht, daß mindestens 90% der Acylgruppen R-CO- 6 bis 18 Kohlenstoffatome enthalten;
b) mindestens ein Sulfosuccinat, dessen Anion die folgende allgemeine Formel besitzt: 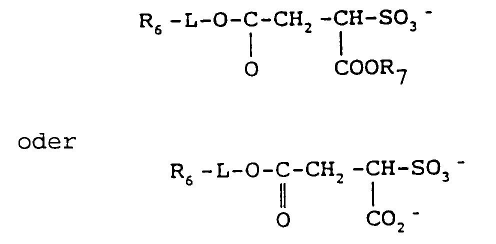 worin einer der Reste R₆ und R₇ unabhängig voneinander für aliphatische Kohlenwasserstoffketten steht, die eine derartige Kettenlängenverteilung aufweisen, daß mindestens 90% der Gruppen R₆ und R₇ 6 bis 18 Kohlenstoffatome enthalten, und wobei L fehlt oder eine verbindende Gruppe ist; und
c) mindestens ein Betain der folgenden allgemeinen Formel: 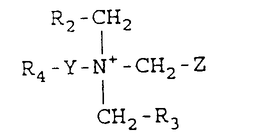 worin R₄ für eine aliphatische Kohlenwasserstoffkette mit einer derartigen Kettenlängenverteilung steht, daß mindestens 90% der Gruppen R₄ 5 bis 17 Kohlenstoffatome enthalten,
R₂ und R₃ unabhängig voneinander für Wasserstoff, Alkyl mit 1 bis 4 Kohlenstoffatom(en) oder Hydroxyalkyl mit 1 bis 4 Kohlenstoffatom(en) stehen, Y CH₂ oder CONHCH₂CH₂CH₂ bedeutet und Z CO₂⁻ oder CHOHCH₂SO₃⁻ entspricht,
und die des weiteren dadurch gekennzeichnet ist, daß entweder
i) L fehlt oder eine Amidogruppe umfaßt, während R₂ und R₃ unabhängig voneinander unter Wasserstoff oder -CH₂OH ausgewählt sind, oder
ii) das Gew.-Verhältnis Acylisethionat zu den anderen Reinigungsmittelwirkstoffen in einem Bereich von 10:1 bis 2:1 liegt,
wobei jedoch eine Zusammensetzung ausgeschlossen ist, bei der es sich um eine wäßrige Flüssigkeit handelt, die 15 Gew.-% eines Reinigungsmittelgemisches enthält, das aus Cocoylisethionat, Kokosnußamidopropylbetain und Dinatriumlaurylethersulfosuccinat mit im Mittel 3 Ethylenoxidresten in einem Gew.-Verhältnis von 50:40:10 besteht.

2. Zusammensetzung nach Anspruch 1 in Riegelform.

3. Zusammensetzung nach Anspruch 1 in flüssiger Form.

4. Zusammensetzung nach Anspruch 1, wobei das Gew.-Verhältnis von Acylisethionat zu den anderen Reinigungsmittelwirkstoffen in einem Bereich von 10:1 bis 2:1 liegt und die Zusammensetzung in Riegelform vorliegt.

5. Zusammensetzung nach Anspruch 1, wobei mindestens 95% der Kohlenstoffkettenverteilung von R zwischen C₈ und C₁₈ liegt und mehr als 30% C₁₄ oder weniger ist.

6. Zusammensetzung nach Anspruch 1, wobei mindestens 95% der Kohlenstoffkettenverteilung von R zwischen C₈ und C₁₈ liegt und mehr als die Hälfte C₁₂ oder weniger ist.

7. Zusammensetzung nach einem der vorhergehenden Ansprüche, wobei das Betain in einer Menge von 0,5 bis 10 Gew.-% der Zusammensetzung vorhanden ist, das Sulfosuccinat in einer Menge von 1 bis 24 Gew.-% der Zusammensetzung vorhanden ist und die Acylester der Isethionsäuresalze in einer derartigen Menge vorhanden sind, daß das Verhältnis Acylisethionate zu den anderen Reinigungsmittelwirkstoffen in einem Bereich von 10:1 bis 2:1 liegt.

8. Zusammensetzung nach einem der vorhergehenden Ansprüche, die des weiteren 1 bis 10 Gew.-% Alkalimetallisethionat umfaßt.

9. Zusammensetzung nach einem der vorhergehenden Ansprüche, wobei das Betain die folgende Formel aufweist:
R₄-CO-NH-CH₂-CH₂-CH₂-N⁺(CH₃)₂·CH₂-COO⁻.

10. Zusammensetzung nach Anspruch 9, wobei mindestens 95% der Kohlenstoffkettenverteilung von R₄ zwischen C₈ und C₁₈ liegt und mehr als die Hälfte C₁₂ oder weniger ist.

11. Zusammensetzung nach einem der vorhergehenden Ansprüche, wobei die Menge an Betain 1 bis 10 Gew.-% der Zusammensetzung ausmacht.

12. Zusammensetzung nach einem der vorhergehenden Ansprüche, wobei das Sulfosuccinat ein Acylmonoethanolaminsulfosuccinat der folgenden Struktur ist:

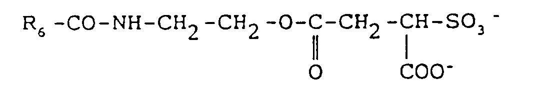

13. Zusammensetzung nach Anspruch 12, wobei mindestens 95% der Kohlenstoffkettenverteilung von R₆ zwischen C₈ und C₁₈ liegt und mehr als die Hälfte C₁₂ oder weniger ist.

14. Zusammensetzung nach einem der vorhergehenden Ansprüche, wobei die Menge an Sulfosuccinat 1 bis 10 Gew.-% der Zusammensetzung beträgt.

15. Zusammensetzung nach einem der vorhergehenden Ansprüche, wobei das Gew.-Verhältnis Betain:Sulfosuccinat in einem Bereich von 2:1 bis 1:10 liegt.

16. Zusammensetzung nach einem der vorhergehenden Ansprüche, wobei das Gew.-Verhältnis Betain:Sulfosuccinat in einem Bereich von 1:1 bis 1:7 liegt.

17. Zusammensetzung nach einem der vorhergehenden Ansprüche, die nicht mehr als 35 Gew.-% Seife enthält.

18. Zusammensetzung nach einem der vorhergehenden Ansprüche, die des weiteren freie Fettsäuren mit etwa 8 bis 22 Kohlenstoffatomen umfaßt.

19. Zusammensetzung nach einem der vorhergehenden Ansprüche, wobei R₂ und R₃ unabhängig voneinander unter Wasserstoff und -CH₂OH ausgewählt sind.

## Revendications

1. Composition de nettoyage de la peau comprenant :
(a) un acyliséthionate dont l'anion répond à la formule générale :
R-CO-O-CH₂CH₂SO₃⁻
dans laquelle R-CO- est un groupe acyle aliphatique avec une distribution de longueurs de chaîne hydrocarbonée telle qu'au moins 90% des groupes acyle R-CO- contiennent 6 à 18 atomes de carbone,
(b) au moins un sulfosuccinate dont l'anion répond à la formule générale : 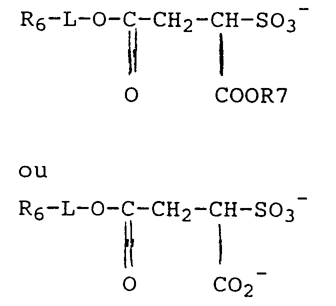 dans laquelle R₆ et R₇ sont indépendamment des chaînes hydrocarbonées montrant une distribution de longueurs de chaîne telle qu'au moins 90% des groupes R₆ et R₇ contiennent 6 à 18 atomes de carbone,
et L est absent ou est un groupe de liaison, et
(c) au moins une bétaine de formule générale : 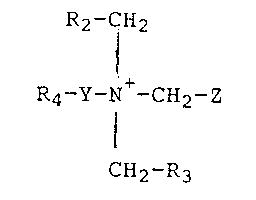 dans laquelle R₄ est de préférence une chaîne hydrocarbonée aliphatique ayant une distribution de longueurs de chaîne telle qu'au moins 90% des groupes R₄ contiennent 5 à 17 atomes de carbone,
R₂ et R₃ sont indépendamment l'hydrogène, alkyle de 1 à 4 atomes de carbone ou hydroxyalkyle de 1 à 4 atomes de carbone,
Y est CH₂ ou CONHCH₂CH₂CH₂ ; et
Z est CO₂⁻ ou CHOHCH₂SO₃- ,
caractérisée de plus en ce que soit :
(i) L est absent ou comporte un groupe amido tandis que R₂ et R₃ sont indépendamment l'hydrogène ou -CH₂OH soit
(ii) le rapport pondéral de l'acyl-iséthionate aux autres détergents actifs est dans la gamme de 10:1 à 2:1.
mais à l'exclusion d'une composition qui est un liquide aqueux contenant 15% en poids d'un mélange détergent consistant en iséthionate de cocoyle, amidopropylbétaine de coprah et lauryléthersulfosuccinate disodique avec une moyenne de 3 résidus oxyde d'éthylène en un rapport pondéral de 50:40:10.

2. Composition selon la revendication 1, qui est sous forme de pain.

3. Composition selon la revendication 1, qui est sous forme liquide.

4. Composition selon la revendication 1, dans laquelle le rapport pondéral de l'acyl-iséthionate aux autres actifs détergents est dans une gamme de 10:1 à 2:1 et la composition est sous forme de pain.

5. Composition selon la revendication 1, dans laquelle au moins 95% de la distribution de chaîne carbonée de R se situent entre C₈ et C₁₈ et plus de 30% sont en C₁₄ ou moins.

6. Composition selon la revendication 1, dans laquelle au moins 95% de la distribution de chaîne carbonée de R se situent entre C₈ et C₁₈ et plus de la moitié sont en C₁₂ ou moins.

7. Composition selon l'une quelconque des revendications précédentes, dans laquelle la bétaine est présente en une quantité de 0,5 à 10% en poids de la composition, le sulfosuccinate est présent en une quantité de 1 à 24% en poids de la composition et les esters acyliques des sels d'acide iséthionique sont présents en une quantité telle que le rapport des acyl-iséthionates aux autres actifs détergents se situe entre 10:1 et 2:1.

8. Composition selon l'une quelconque des revendications précédentes, qui comprend en plus de 1 à 10% en poids d'iséthionate de métal alcalin.

9. Composition selon l'une quelconque des revendications précédentes, dans laquelle la bétaine répond à la formule :
R₄-CO-NH-CH₂-CH₂-CH₂-N⁺(CH₃)₂·CH₂-COO⁻

10. Composition selon la revendication 9, dans laquelle au moins 95% de la distribution de chaîne carbonée de R₄ se situent entre C₈ et C₁₈ et plus de la moitié sont en C₁₂ ou moins.

11. Composition selon l'une quelconque des revendications précédentes, dans laquelle la quantité de bétaine est de 1 à 10% en poids de la composition.

12. Composition selon l'une quelconque des revendications précédentes, dans laquelle le sulfosuccinate est le monoéthanolamine-sulfosuccinate acylique de structure :

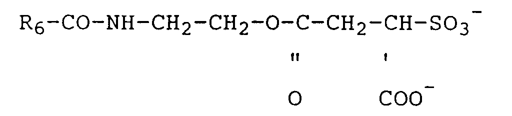

13. Composition selon la revendication 12, dans laquelle au moins 95% de la distribution de chaîne carbonée de R₆ se situent entre C₈ et C₁₈ et plus de la moitié sont en C₁₂ ou moins.

14. Composition selon l'une quelconque des revendications précédentes, dans laquelle la quantité de sulfosuccinate est de 1 à 10% en poids de la composition.

15. Composition selon l'une quelconque des revendications précédentes, dans laquelle le rapport pondéral de la bétaine au sulfosuccinate est dans la gamme des 2:1 à 1:10.

16. Composition selon l'une quelconque des revendications précédentes, dans laquelle le rapport pondéral de la bétaine au sulfosuccinate est dans la gamme des 1:1 à 1:7.

17. Composition selon l'une quelconque des revendications précédentes, qui ne contient pas plus de 35% en poids de savon.

18. Composition selon l'une quelconque des revendications précédentes, comprenant de plus des acides gras libres de 8 à 22 atomes de carbone.

19. Composition selon l'une quelconque des revendications précédentes, dans laquelle R₂ et R₃ sont indépendamment l'hydrogène ou -CH₂OH.
